(19) Europäisches Patentamt
European Patent Office
Office européen des brevets

(11) **EP 3 771 404 A1**

(12) **EUROPEAN PATENT APPLICATION**

(43) Date of publication:
**03.02.2021 Bulletin 2021/05**

(21) Application number: **19189368.4**

(22) Date of filing: **31.07.2019**

(51) Int Cl.:
***A61B 5/0402*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **BONOMI, Alberto Giovanni**
**5656 AE Eindhoven (NL)**
• **HUIJBREGTS, Laurentia Johanna**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **CALIBRATING A SENSOR**

(57)    According to an aspect there is provided an apparatus for calibrating a sensor of a wearable device to a reference frame of a subject on which the wearable device is to be worn. The wearable device comprises an electrocardiogram, ECG, electrode arrangement comprising three or more ECG electrodes that are in a predefined arrangement with respect to each other, and the sensor has a measurement axis in a predefined orientation with respect to the ECG electrode arrangement. The apparatus comprises a processing unit configured to determine an orientation of the ECG electrode arrangement in the reference frame of the subject based on ECG signals obtained by respective pairs of the ECG electrodes; and determine a relationship between the measurement axis of the sensor and the reference frame of the subject based on the determined orientation of the ECG electrode arrangement in the reference frame of the subject and the predefined orientation of the measurement axis with respect to the ECG electrode arrangement.

Fig. 1

EP 3 771 404 A1

**Description**

FIELD OF THE INVENTION

The disclosure relates to an apparatus, system and a computer-implemented method for calibrating a sensor that is in a wearable device.

BACKGROUND OF THE INVENTION

Wearable devices that include one or more sensors can be used to measure vital signs, activity and posture of a patient at home or during a hospital stay. For instance, accelerometers are small and inexpensive sensors that can measure acceleration with respect to the Earth's gravitational field. As such, accelerometers are widely used to measure movements and orientation. When used in combination with a dedicated algorithm, an accelerometer placed on a body part can be used to recognize certain activities such as lying in bed, sitting, standing, walking upright. Information on the orientation of the accelerometer sensing axes with respect to the body anthropometric directions is necessary in order to determine the posture of a patient and characterise the orientation of the body during certain activities like walking. For example, the angle between the gravity vector sensed by the accelerometer and the anthropometric axis can be measured to determine whether the patient is lying, upright, or reclining.

Determining the body posture (e.g. standing up, lying prone, lying supine, lying on their side) and activity information is relevant for a medical professional (e.g. a clinician, nurse, etc.) to assess patient status during a recovery process in hospital or at home following injury or surgery, for example. Information on body posture has several uses. For example, the correlation between vital signs or symptoms and the angle of recline in bed-bound patients is important to assess heart failure and respiratory disease severity. Patients that are able to stay more supine are usually less compromised than those requiring an upright posture in bed. Furthermore, in order to reduce risk of pressure ulcers, a posture of a patient needs to be accurately and carefully monitored.

Wearable devices having one or more sensors are usually positioned in a single location on the body of the patient. For example, a wearable device aimed at collecting electrocardiogram (ECG) measurements and body acceleration measurements may be positioned in a designated location of the body close to the heart on the chest area. However, the procedure to attach a wearable device to the body of the patient is difficult to standardise and it is up to the discretion of the medical professional (or the patient themselves in the home environment) to decide which location is best for a patient, e.g. given heterogeneity in body shape and skin condition. This means that the orientation of the wearable device is often arbitrary and subject to change depending on the patient and the operator. Furthermore, the posture of the patient during application of the wearable sensor is also unknown by the wearable device. This means that, for measurements from the sensor to be analysed or interpreted correctly (e.g. acceleration measurements from an accelerometer), it is necessary to determine the orientation of the sensor/wearable device relative to the patient's frame of reference or relative to a global (e.g. the Earth's) frame of reference.

There exist methods for determining an orientation of a wearable device on a subject. For example, WO2017/191036 discloses that characteristics of physiological characteristic signals acquired from a sensor of a wearable device differ depending on the position and/or orientation of the wearable device on the subject and thus the orientation of the wearable device on the subject can be determined based on one or more characteristics of a physiological characteristic signal. The one or more characteristics of the physiological characteristic signal are compared to a set of characteristics that are stored in a memory with corresponding orientations in order for the orientation of the wearable device on the subject to be determined. However, there are an unlimited number of orientations of the sensor/wearable device on the patient that are possible in practice, and so an improved method of determining an orientation of a wearable device/sensor on a patient for calibrating the sensor would be valuable.

SUMMARY OF THE INVENTION

As noted above, the limitations with existing techniques is that it is only possible to clearly distinguish between a predetermined number of orientations and thus it is not possible to calibrate a sensor irrespective of the orientation in which the sensor is placed. It would therefore be valuable to have an improvement aimed at addressing these limitations.

Therefore, according to a first aspect, there is provided an apparatus for calibrating a sensor of a wearable device to a reference frame of a subject on which the wearable device is to be worn, the wearable device comprising an electrocardiogram, ECG, electrode arrangement comprising three or more ECG electrodes that are in a predefined arrangement with respect to each other, and the sensor having a measurement axis in a predefined orientation with respect to the ECG electrode arrangement. The apparatus comprises a processing unit configured to: determine an orientation of the ECG electrode arrangement in the reference frame of the subject based on ECG signals obtained by respective pairs of the ECG electrodes; and determine a relationship between the measurement axis of the sensor and the reference frame of the subject based on the determined orientation of the ECG electrode arrangement in the reference frame of the subject and the predefined orientation of the measurement axis with respect to the ECG electrode arrangement. Thus, the apparatus implements an improved technique in which the orientation of a wearable device is determined based on ECG signals, and the sensor is calibrated based on that determined orientation.

In some embodiments the reference frame of the subject includes a reference axis relating to the heart of the subject, with the reference axis relating to a predefined ECG signal characteristic, and the processing unit is configured to determine the orientation of the ECG electrode arrangement by: identifying an ECG electrode pair in the ECG electrode arrangement for which the ECG signal obtained by the ECG electrode pair meets a criterion relating to the predefined ECG signal characteristic; and determining the orientation of the ECG electrode arrangement with respect to the reference frame of the subject based on a first virtual axis defined by the ECG electrodes in the identified ECG electrode pair and the relation between the reference axis and the predefined ECG signal characteristic. Thus the orientation of the ECG electrode arrangement is determined by making use of a reference axis and corresponding ECG signal characteristic of the heart, and as the heart has a relatively similar orientation across different subjects, the ECG signals from the heart can be used as a consistent reference for determining the orientation of the ECG electrode arrangement.

In alternative embodiments, the reference frame of the subject includes a reference axis relating to the heart of the subject, wherein the reference axis is related to a predefined ECG signal characteristic, and wherein the processing unit is configured to determine the orientation of the ECG electrode arrangement by: identifying two adjacent ECG electrode pairs in the ECG electrode arrangement for which the ECG signals obtained by the two adjacent ECG electrode pairs best meet a criterion relating to the predefined ECG signal characteristic; determining a first virtual axis as a virtual axis between a virtual axis defined by the ECG electrodes in a first one of the identified ECG electrode pairs and a virtual axis defined by the ECG electrodes in the other one of the identified ECG electrode pairs; and determining the orientation of the ECG electrode arrangement with respect to the reference frame of the subject based on the first virtual axis and the relation between the reference axis and the predefined ECG signal characteristic. These embodiments have similar benefits to the previous embodiments, and also have the benefit that the orientation of the ECG electrode arrangement can be determined more accurately as by interpolating between the two ECG electrode pairs that best meet the criterion.

In either set of embodiments, the predefined ECG signal characteristic can be the QRS complex and the criterion can be met by the ECG signal that has the smallest QRS complex; or the smallest difference in maximum voltage amplitude and minimum voltage amplitude.

In these embodiments, the reference axis can be a QRS axis of the heart of the subject, and the first virtual axis can be considered to be perpendicular or substantially perpendicular to the QRS axis.

In alternative embodiments, the predefined ECG signal characteristic can be the R-peak and the criterion can be met by the ECG signal that has the largest R-peak; or the largest difference in maximum voltage amplitude and minimum voltage amplitude.

In these embodiments, the reference axis can be a QRS axis of the heart of the subject, and the first virtual axis can be considered to be parallel to the QRS axis.

In either of the embodiments where the predefined ECG signal characteristic can be the QRS complex or the R-peak, the processing unit can be further configured to determine a polarity of the ECG signal obtained by the identified ECG electrode pair to estimate a direction of the QRS axis and determine the orientation of the ECG electrode arrangement with respect to the left and right side of the subject. These embodiments have the benefit that it is possible to determine the orientation of the ECG electrode arrangement with respect to the left and right side of the subject just based on the ECG signals.

In these embodiments the processing unit can be further configured to determine the relationship between the measurement axis of the sensor and the reference frame of the subject based on the determined orientation of the ECG electrode arrangement with respect to the left and right side of the subject.

In some embodiments the orientation of the reference axis in the reference frame of the subject is dependent on one or more physiological characteristics of the subject and/or clinical information on the subject. These embodiments provide that the orientation of the reference axis for the subject can be adjusted based on their specific physiology, for example if their heart is at an unusual or atypical angle in their body.

In some embodiments the processing unit is further configured to determine the relationship between the measurement axis of the sensor and the reference frame of the subject based on a shape of the part of the body of the subject on which the wearable device is to be worn. These embodiments have the benefit that the sensor can be calibrated more accurately by accounting for different body shapes of different subjects, and/or by accounting for the different orientations of the wearable device that can arise for a particular user depending on the part of their body on which the wearable device is being worn.

In some embodiments the determined relationship between the measurement axis of the sensor and the reference frame of the subject is a rotation required to (i) rotate measurements obtained by the sensor in the reference frame of the sensor into the reference frame of the subject and/or (ii) rotate one or more parameters or rules defined in the reference frame of the subject into the reference frame of the sensor.

In these embodiments the processing unit can be configured to: acquire measurements from the sensor; apply the rotation to the acquired measurements to rotate the acquired measurements into the reference frame of the subject; and evaluate the rotated measurements to determine the posture of the subject using one or more parameters and/or rules defined with respect to the reference frame of the subject.

In alternative embodiments, the processing unit can be configured to: acquire measurements from the sensor; apply the rotation to one or more parameters and/or rules defined with respect to the reference frame of the subject to rotate the one or more parameters and/or rules into the reference frame of the sensor; and evaluate the acquired measurements to determine the posture of the subject using the rotated one or more parameters and/or rules.

In some embodiments the sensor is an accelerometer.

According to a second aspect, there is provided a system for calibrating a sensor of a wearable device to a reference frame of a subject on which the wearable device is worn. The system comprises the apparatus according to the first aspect or any embodiment thereof; and the wearable device comprising the ECG electrode arrangement and the sensor having the measurement axis in the predefined orientation with respect to the ECG electrode arrangement.

In some embodiments the apparatus is part of the wearable device. In alternative embodiments the apparatus is separate from the wearable device.

According to a third aspect, there is provided a method of calibrating a sensor of a wearable device to a reference frame of a subject on which the wearable device is to be worn, the wearable device comprising an electrocardiogram, ECG, electrode arrangement comprising three or more ECG electrodes that are in a predefined arrangement with respect to each other, and the sensor having a measurement axis in a predefined orientation with respect to the ECG electrode arrangement. The method comprises determining an orientation of the ECG electrode arrangement in the reference frame of the subject based on ECG signals obtained by respective pairs of the ECG electrodes; and determining a relationship between the measurement axis of the sensor and the reference frame of the subject based on the determined orientation of the ECG electrode arrangement in the reference frame of the subject and the predefined orientation of the measurement axis with respect to the ECG electrode arrangement. Thus, the method provides an improved technique in which the orientation of a wearable device is determined based on ECG signals, and the sensor is calibrated based on that determined orientation.

In some embodiments the reference frame of the subject includes a reference axis relating to the heart of the subject, with the reference axis relating to a predefined ECG signal characteristic, and the step of determining the orientation of the ECG electrode arrangement comprises identifying an ECG electrode pair in the ECG electrode arrangement for which the ECG signal obtained by the ECG electrode pair meets a criterion relating to the predefined ECG signal characteristic; and determining the orientation of the ECG electrode arrangement with respect to the reference frame of the subject based on a first virtual axis defined by the ECG electrodes in the identified ECG electrode pair and the relation between the reference axis and the predefined ECG signal characteristic. Thus the orientation of the ECG electrode arrangement is determined by making use of a reference axis and corresponding ECG signal characteristic of the heart, and as the heart has a relatively similar orientation across different subjects, the ECG signals from the heart can be used as a consistent reference for determining the orientation of the ECG electrode arrangement.

In alternative embodiments, the reference frame of the subject includes a reference axis relating to the heart of the subject, wherein the reference axis is related to a predefined ECG signal characteristic, and wherein the step of determining the orientation of the ECG electrode arrangement comprises: identifying two adjacent ECG electrode pairs in the ECG electrode arrangement for which the ECG signals obtained by the two adjacent ECG electrode pairs best meet a criterion relating to the predefined ECG signal characteristic; determining a first virtual axis as a virtual axis between a virtual axis defined by the ECG electrodes in a first one of the identified ECG electrode pairs and a virtual axis defined by the ECG electrodes in the other one of the identified ECG electrode pairs; and determining the orientation of the ECG electrode arrangement with respect to the reference frame of the subject based on the first virtual axis and the relation between the reference axis and the predefined ECG signal characteristic. These embodiments have similar benefits to the previous embodiments, and also have the benefit that the orientation of the ECG electrode arrangement can be determined more accurately as by interpolating between the two ECG electrode pairs that best meet the criterion.

In either set of embodiments, the predefined ECG signal characteristic can be the QRS complex and the criterion can be met by the ECG signal that has the smallest QRS complex; or the smallest difference in maximum voltage amplitude and minimum voltage amplitude.

In these embodiments, the reference axis can be a QRS axis of the heart of the subject, and the first virtual axis can be considered to be perpendicular or substantially perpendicular to the QRS axis.

In alternative embodiments, the predefined ECG signal characteristic can be the R-peak and the criterion can be met by the ECG signal that has the largest R-peak; or the largest difference in maximum voltage amplitude and minimum voltage amplitude.

In these embodiments, the reference axis can be a QRS axis of the heart of the subject, and the first virtual axis can be considered to be parallel to the QRS axis.

In either of the embodiments where the predefined ECG signal characteristic can be the QRS complex or the R-peak, the method can further comprise determining a polarity of the ECG signal obtained by the identified ECG electrode pair to estimate a direction of the QRS axis and determine the orientation of the ECG electrode arrangement with respect to the left and right side of the subject. These embodiments have the benefit that it is possible to determine the orientation of the ECG electrode arrangement with respect to the left and right side of the subject just based on the ECG signals.

In these embodiments the step of determining the relationship between the measurement axis of the sensor and the reference frame of the subject can comprise determining the relationship between the measurement axis of the sensor and the reference frame of the subject based on the determined orientation of the ECG electrode arrangement with respect to the left and right side of the subject.

In some embodiments the orientation of the reference axis in the reference frame of the subject is dependent on one or more physiological characteristics of the subject and/or clinical information on the subject. These embodiments provide that the orientation of the reference axis for the subject can be adjusted based on their specific physiology, for example if their heart is at an unusual or atypical angle in their body.

In some embodiments the step of determining the relationship between the measurement axis of the sensor and the reference frame of the subject comprises determining the relationship between the measurement axis of the sensor and the reference frame of the subject based on a shape of the part of the body of the subject on which the wearable device is to be worn. These embodiments have the benefit that the sensor can be calibrated more accurately by accounting for different body shapes of different subjects, and/or by accounting for the different orientations of the wearable device that can arise for a particular user depending on the part of their body on which the wearable device is being worn.

In some embodiments the determined relationship between the measurement axis of the sensor and the reference frame of the subject is a rotation required to (i) rotate measurements obtained by the sensor in the reference frame of the sensor into the reference frame of the subject and/or (ii) rotate one or more parameters or rules defined in the reference frame of the subject into the reference frame of the sensor.

In these embodiments the method can further comprise: acquiring measurements from the sensor; applying the rotation to the acquired measurements to rotate the acquired measurements into the reference frame of the subject; and evaluating the rotated measurements to determine the posture of the subject using one or more parameters and/or rules defined with respect to the reference frame of the subject.

In alternative embodiments, the method can further comprise: acquiring measurements from the sensor; applying the rotation to one or more parameters and/or rules defined with respect to the reference frame of the subject to rotate the one or more parameters and/or rules into the reference frame of the sensor; and evaluating the acquired measurements to determine the posture of the subject using the rotated one or more parameters and/or rules.

In some embodiments the sensor is an accelerometer.

According to a fourth aspect, there is provided a computer program product comprising a computer readable medium. The computer readable medium has computer readable code embodied therein. The computer readable code is configured such that, on execution by a suitable computer or processor, the computer or processor is caused to perform the method described above.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a block diagram illustrating a system comprising an apparatus and wearable device according to an exemplary embodiment;
Fig. 2 shows three exemplary arrangements of ECG electrodes;
Fig. 3 is a flow chart illustrating a method according to an embodiment;
Fig. 4 is an illustration of the ECG electrode arrangement of Fig. 2(c) in a first orientation on a body of a subject and the corresponding ECG signals obtained by the ECG electrode arrangement;
Fig. 5 is an illustration of the ECG electrode arrangement of Fig. 2(c) in a second orientation on the body of the subject and the corresponding ECG signals obtained by the ECG electrode arrangement; and
Fig. 6 is a flow chart illustrating a general method according to the techniques described herein.

DETAILED DESCRIPTION OF EMBODIMENTS

As noted above, there is provided herein an improved technique for calibrating a sensor that is part of a wearable device. Briefly, the improved technique uses electrocardiogram (ECG) measurements of a subject (e.g. patient) by a plurality of ECG electrodes on the wearable device to determine the orientation of the ECG electrodes (and thus the orientation of the wearable device) on the subject, and determines a relationship between the orientation of the sensor and the orientation of the subject from the determined orientation of the ECG electrodes and a predefined orientation of the sensor with respect to the ECG electrodes.

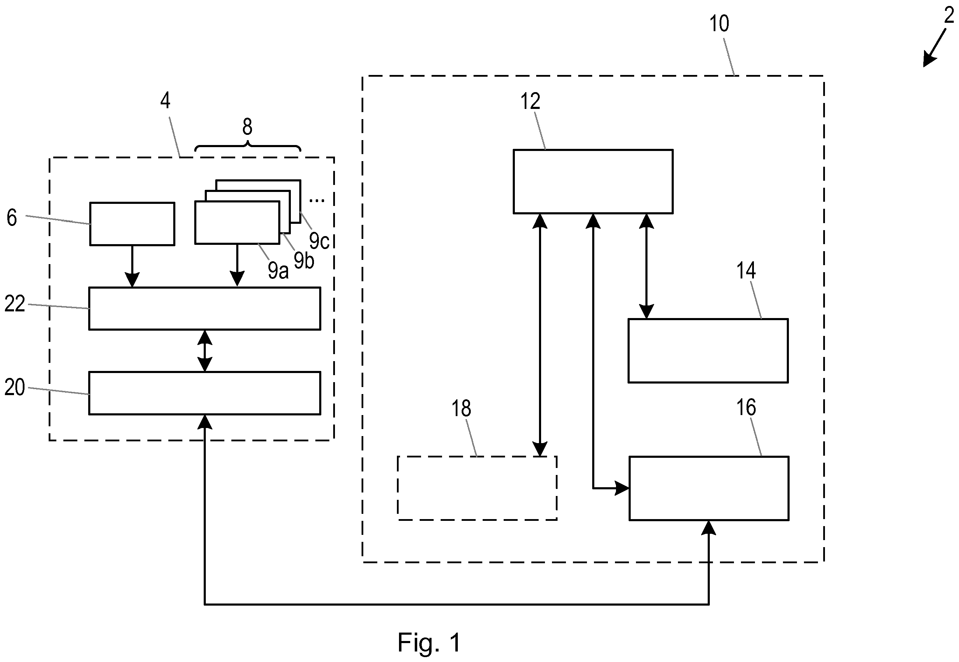

Fig. 1 illustrates a system 2 according to an exemplary embodiment of the teachings presented herein. In this embodiment the system 2 comprises a wearable device 4 that is to be carried or worn by the subject and that includes a sensor 6 for measuring the subject (e.g. the subject's movement, including the acceleration of the subject) and/or an aspect of the environment around the subject (e.g. air pressure) over time. The sensor 6 provides measurements relative to at least one measurement axis (e.g. an x-axis, y-axis and/or z-axis in the case of an accelerometer or similar).

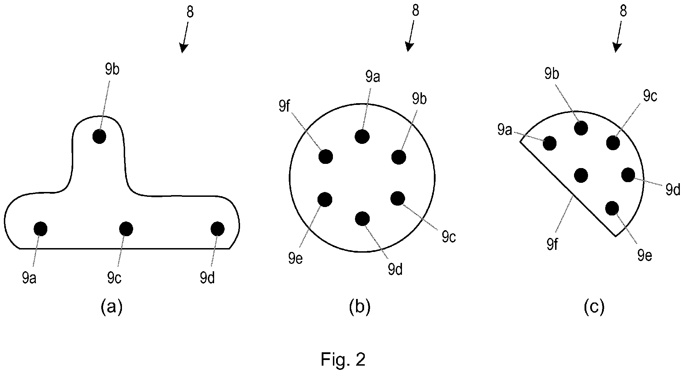

The wearable device 4 also includes an ECG electrode arrangement 8 that comprises a plurality of ECG electrodes 9 (and specifically at least three ECG electrodes 9) that are for obtaining ECG signals for the subject. In Fig. 1 three ECG electrodes 9 are shown that are respectively labelled 9a, 9b, 9c, .... The ECG electrodes 9 are in a predefined (i.e. fixed) arrangement with respect to each other and are fixed within or on the wearable device 4 so that the ECG electrodes 9 have a predefined orientation with respect to the wearable device 4. The ECG electrodes 9 enable multiple ECG signals to be obtained from the subject, with each 'ECG lead' (as defined by a virtual axis interconnecting a pair of the ECG electrodes 9) having a respective orientation with respect to the wearable device 4, and thus a respective orientation with respect to the subject when the wearable device 4 is being carried or worn by the subject. Some exemplary ECG electrode arrangements 8 are described below with reference to Fig. 2.

The sensor 6 is fixed within or on the wearable device 4, which means that the orientation of the sensor 6 (and particular an orientation of a measurement axis of the sensor 6) is fixed with respect to the wearable device 4 and fixed with respect to the ECG electrode arrangement 8. The orientation of the sensor 6 with respect to the orientation of the ECG electrode arrangement 8 is known.

The sensor 6 generates and outputs a respective signal representing measurements of the subject or environment over time. Each measurement signal can comprise a time series of measurements (samples), and the measurement signal can therefore relate to the measurements in a time period. The sensor 6 can use any desired sampling frequency, for example 50 measurements per second (50 Hz), 64 Hz or 100 Hz. Different sensors may run at different sampling rates. For example, another sensor may be sampled at 2 Hz, or 4Hz, or 0.4 Hz, or 1 Hz.

In some embodiments, the sensor 6 in the system is a sensor that may directly or indirectly measure the movements of the subject over time. For example, the sensor 6 can be any of an accelerometer, an air pressure sensor, a magnetometer, or a gyroscope. In preferred embodiments the sensor 6 is an accelerometer. The measurement signal from the sensor 6 may be analysed to determine the posture of the subject, identify and/or evaluate the movement of the subject.

In the case of the sensor 6 being an accelerometer, the accelerometer can generate and output a measurement signal that contains a plurality of acceleration measurement samples representing the movements of the subject at a plurality of time instants. The accelerometer is typically an accelerometer that measures accelerations in three dimensions, and the measurement signal generated by the accelerometer can include respective measurements representing the accelerations in each of the three dimensions. For example, the output of the accelerometer can include respective measurement signals for one or more of three measurement axes, e.g. an x-axis, y-axis and z-axis of a Cartesian coordinate system.

In the case of the sensor 6 being a gyroscope, the gyroscope can generate and output a measurement signal that contains a plurality of gyroscope measurement samples representing the rotational movement and/or orientation of the wearable device at a plurality of time instants.

In the case of the sensor 6 being an air pressure sensor, the air pressure sensor can include any type of sensor for measuring air pressure or changes in air pressure. The air pressure sensor can generate and output a measurement signal representing measurements of air pressure or changes in air pressure at the air pressure sensor. The measurement signal can comprise a time series of air pressure measurements (samples) and the measurement signal can therefore relate to the air pressure or changes in air pressure in a time period. The air pressure sensor can use any desired sampling frequency, for example 1 Hz or 50 Hz. In other embodiments a microphone might be used. Typically, the microphone is sampled at 16 kHz or higher frequencies.

Returning to Fig. 1, the system 2 also comprises an apparatus 10 that receives the ECG signals and that analyses the ECG signals to enable measurements by the sensor 6 to be calibrated to the reference frame of the subject. In some embodiments the apparatus 10 may also receive the measurement signal(s) from the sensor 6 and use the determined calibration to, e.g. analyse the movement or physical activity of the subject, determine the posture of the subject, identify and/or evaluate the movement of the subject.

The wearable device 4 can be in any form suitable enabling the subject to carry or wear the device 4, and that is able to obtain ECG signals from the subject. In particular the wearable device 4 can be worn or carried on the torso or chest (e.g. near to the heart), or on the neck. For example, the wearable device 4 may be in the form of a pendant, a necklace, an adhesive patch, a chest band, integrated into an item of clothing, etc. In some embodiments, as shown in Fig. 1, the apparatus 10 can be separate from the wearable device 4. In these embodiments, the apparatus 10 can be any type of electronic device or computing device that can communicate with, or otherwise receive the ECG signals and optionally the measurement signal(s) directly or indirectly from, the wearable device 4. For example the apparatus 10 can be, or be part of, a computer, a laptop, a tablet, a smartphone, a smartwatch, etc., and as such may be an apparatus that is present or used in the home or care environment of the subject. In other implementations, the apparatus 10 can be an apparatus that is remote from the subject, and remote from the home or care environment of the subject. For example, the apparatus 10 can be a server, for example a server in a data centre (also referred to as being 'in the cloud'). In alternative embodiments, the apparatus 10 (and in particular the functionality of the apparatus 10 as described herein) can be integral with the wearable device 4. Therefore the apparatus 10 can also be carried or worn by the subject as part of the wearable device 4.

The apparatus 10 includes a processing unit 12 that controls the operation of the apparatus 10 and that can be configured to execute or perform the methods described herein. In particular, the processing unit 12 can obtain the ECG signals and process them to determine a relationship between a or the measurement axis of the sensor 6 and the reference frame of the subject. The processing unit 12 can be implemented in numerous ways, with software and/or hardware, to perform the various functions described herein. The processing unit 12 may comprise one or more microprocessors or digital signal processor (DSPs) that may be programmed using software or computer program code to perform the required functions and/or to control components of the processing unit 12 to effect the required functions. The processing unit 12 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 12 is connected to a memory unit 14 that can store data, information and/or signals (including ECG signals) for use by the processing unit 12 in controlling the operation of the apparatus 10 and/or in executing or performing the methods described herein. For example the memory unit 14 can store information on the predefined arrangement of the ECG electrodes 9 with respect to each other, and/or information on the orientation of the sensor 6 with respect to the orientation of the ECG electrode arrangement 8 (e.g. in terms of an angle in 2-dimensional (2D) or 3D space between the measurement axis of the sensor 6 and the orientation(s) of the ECG leads formed by the ECG electrodes 9 in the ECG electrode arrangement 8. In some implementations the memory unit 14 stores computer-readable code that can be executed by the processing unit 12 so that the processing unit 12 performs one or more functions, including the methods described herein. In particular embodiments, the program code can be in the form of an application for a smartwatch, a smartphone, tablet, laptop or computer. The memory unit 14 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In the embodiment of the system 2 shown in Fig. 1, as the apparatus 10 is separate from the wearable device 4 that includes the sensor 6 and ECG electrode arrangement 8, the apparatus 10 also includes interface circuitry 16 for enabling a data connection to and/or data exchange with other devices, including wearable device 4, and optionally any one or more of servers, databases, user devices, and other sensors. The connection may be direct or indirect (e.g. via the Internet), and thus the interface circuitry 16 can enable a connection between the apparatus 10 and a network, such as the Internet, or between the apparatus 10 and wearable device 4, via any desirable wired or wireless communication protocol. For example, the interface circuitry 16 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 16 (and thus apparatus 10) may include one or more suitable antennas for transmitting/receiving over a transmission medium (e.g. the air). Alternatively, in the case of a wireless connection, the interface circuitry 16 may include means (e.g. a connector or plug) to enable the interface circuitry 16 to be connected to one or more suitable antennas external to the apparatus 10 for transmitting/receiving over a transmission medium (e.g. the air). The interface circuitry 16 is connected to the processing unit 12 to enable information or data received by the interface circuitry 16 to be provided to the processing unit 12, and/or information or data from the processing unit 12 to be transmitted by the interface circuitry 16.

The interface circuitry 16 can be used to receive the ECG signals from the ECG electrode arrangement 8 and optionally also the measurements/measurement signal generated by the sensor 6.

In some embodiments, the interface circuitry 16 can be used to output a result of the processing by the processing unit 12, for example an indication of the determined relationship between a measurement axis of the sensor 6 and the reference frame of the subject.

In some embodiments, the apparatus 10 comprises a user interface 18 that includes one or more components that enables a user of apparatus 10 (e.g. the subject, or a care provider for the subject) to input information, data and/or commands into the apparatus 10 (e.g. for starting or enabling the technique of calibrating the sensor 6 to the reference frame of the subject according to the techniques described herein), and/or enables the apparatus 10 to output information or data to the subject or other user of the apparatus 10. The user interface 18 can comprise any suitable input component(s), including but not limited to a keyboard, keypad, one or more buttons, switches or dials, a mouse, a track pad, a touchscreen, a stylus, a camera, a microphone, etc., and the user interface 18 can comprise any suitable output component(s), including but not limited to a display screen, one or more lights or light elements, one or more loudspeakers, a vibrating element, etc.

It will be appreciated that a practical implementation of apparatus 10 may include additional components to those shown in Fig. 1. For example the apparatus 10 may also include a power supply, such as a battery, or components for enabling the apparatus 10 to be connected to a mains power supply.

As noted above, the sensor 6 and ECG electrode arrangement 8 are part of wearable device 4, which is separate from the apparatus 10 in the embodiment shown in Fig. 1. In order for the ECG signals to be communicated from the wearable device 4 to the apparatus 10, the wearable device 4 comprises interface circuitry 20. The interface circuitry 20 may be implemented in a similar way to the interface circuitry 16 in the apparatus 10.

In some embodiments, the wearable device 4 can also include a processing unit 22 for controlling the operation of the wearable device 4. This processing unit 22 can also be used to perform some pre-processing of the ECG signals and/or measurement signal(s) from the sensor 6 before they are communicated to the apparatus 10, for example the ECG signals and/or measurement signal can be filtered to reduce or remove a noise component or artefacts. The processing unit 22 may be implemented in a similar way to the processing unit 12 in the apparatus 10.

It will be appreciated that a practical implementation of wearable device 4 may include additional components to those shown in Fig. 1. For example the wearable device 4 may also include a power supply, preferably a battery so that the device 4 is portable, or components for enabling the wearable device 4 to be connected to a mains power supply.

In alternative embodiments of the system 2 where the apparatus 10 is part of the wearable device 4, it will be appreciated that only one processing unit 12/22 may be present, and interface circuitry is not required to communicate the measurements/signal(s) to the processing unit 12.

As noted above, the ECG electrodes 9 are in a predefined (i.e. fixed) arrangement with respect to each other to enable multiple ECG signals to be obtained from the subject. The ECG electrodes 9 in the ECG electrode arrangement 8 may be arranged symmetrically or asymmetrically. The ECG electrode arrangement 8 may have one or more ECG electrodes 9 that act as 'common' or 'reference' ECG electrodes 9 and are used for obtaining multiple ECG signals in turn with other ones of the ECG electrodes 9, or each ECG electrode 9 in the ECG electrode arrangement 8 may be part of a respective pair of ECG electrodes 9. The ECG electrode arrangements 8 described herein are also known as multiple short-lead ECG electrode arrangements. Three exemplary ECG electrode arrangements 8 are shown in Fig. 2(a), 2(b) and 2(c).

The ECG electrode arrangement 8 shown in Fig. 2(a) comprises four ECG electrodes 9, labelled 9a, 9b, 9c and 9d. In this ECG electrode arrangement 8, ECG electrode 9c is used as a common/reference ECG electrode in conjunction with each of ECG electrodes 9a, 9b and 9d. Thus a first ECG signal can be obtained using a first ECG lead formed by the electrode pair 9c and 9a, a second ECG signal can be obtained using a second ECG lead formed by the electrode pair 9c and 9b, and a third ECG signal can be obtained using a third ECG lead formed by the electrode pair 9c and 9d. The arrangement of the ECG electrodes 9a-d with respect to each other is such that the first ECG lead and the third ECG lead are generally in line with each other, but the second ECG lead is generally oriented perpendicularly to the first ECG lead and the third ECG lead.

The ECG electrode arrangement 8 shown in Fig. 2(b) comprises six ECG electrodes 9, labelled 9a-f. In this ECG electrode arrangement 8, the ECG electrodes 9 are generally arranged in a circle, with each ECG electrode 9 being paired with an ECG electrode 9 that is diametrically opposite in the arrangement. Thus a first ECG signal can be obtained using a first ECG lead formed by the electrode pair 9a and 9d, a second ECG signal can be obtained using a second ECG lead formed by the electrode pair 9b and 9e, and a third ECG signal can be obtained using a third ECG lead formed by the electrode pair 9c and 9f. As the ECG electrodes 9 are generally evenly spaced apart, this arrangement of the ECG electrodes means that each ECG lead differs in orientation from the neighbouring ECG lead by around 60°.

The ECG electrode arrangement 8 shown in Fig. 2(c) comprises six ECG electrodes 9, labelled 9a-f. In this ECG electrode arrangement 8, ECG electrode 9f is used as a common/reference ECG electrode in conjunction with each of ECG electrodes 9a-e which are arranged radially around the common/reference ECG electrode 9f. Thus a first ECG signal can be obtained using a first ECG lead formed by the electrode pair 9f and 9a, a second ECG signal can be obtained using a second ECG lead formed by the electrode pair 9f and 9b, a third ECG signal can be obtained using a third ECG lead formed by the electrode pair 9f and 9c, a fourth ECG signal can be obtained using a fourth ECG lead formed by the electrode pair 9f and 9d and a fifth ECG signal can be obtained using a fifth ECG lead formed by the electrode pair 9f and 9e. The ECG electrodes 9a-e are arranged around ECG electrode 9f such that the neighbouring ECG leads are oriented around 36° (180°/5) apart.

It will be appreciated that the ECG electrodes 9a-f in Fig. 2(b) and the ECG electrodes 9a-e in Fig. 2(c) are spaced equally apart (both in terms of distance between adjacent ECG electrodes 9 and the distance between the ECG electrodes 9 in an electrode pair, which can make the comparison of ECG signals obtained by the ECG leads easier. However, it is possible for the distances between the ECG electrodes 9 to be unequal, although these differences should be taken into account when analysing the ECG signals from the ECG electrodes 9.

As the two ECG electrode arrangements 8 shown in Figs. 2(a) and 2(c) are asymmetrical, it will be appreciated that it may be necessary or useful for these ECG electrode arrangements 8 to be worn or carried on the subject in a particular orientation or in an orientation in a particular range of orientations. This or these orientations will become apparent from the discussion below. The ECG electrode arrangement 8 shown in Fig. 2(b) is symmetrical, so there may not be any limitations on the orientation that the ECG electrode arrangement 8 is carried or worn on the subject.

As noted above the techniques described herein provide a solution for automatically identifying the orientation of the wearable device 4 and thus the orientation of the sensor 6 in the wearable device 4 based on ECG signals obtained by the ECG electrode arrangement 8. In various embodiments the ECG signals obtained by the ECG electrode arrangement 8 are used to identify the direction of the so-called "QRS axis", which corresponds to the segment of the heart connecting the atrio-ventricular node and the apex of the cardiac ventricle. The QRS axis has a known orientation in the body of the subject (with that orientation being a standard value derived from a population of subjects, or it can be specific to the subject) and so information on the orientation of the ECG electrode arrangement 8 with respect to the QRS axis enables the orientation of the ECG electrode arrangement 8 with respect to other axes in the reference frame of the subject to be determined, such as the caudo-cranial direction of the body which is the axis running from tail to head of the subject's body. For example, for an average subject, the QRS axis 54 is typically 38° left from the caudo-cranial axis. The known orientation of the measurement axis of the sensor 6 with respect to the ECG electrode arrangement 8, and the determined orientation of the ECG electrode arrangement 8 with respect to the reference frame of the subject enables a relationship to be determined between a measurement axis of the sensor 6 and the reference frame of the subject.

In the following discussion, the sensor 6 is an accelerometer, but it will be appreciated that the technique is also applicable to embodiments where the sensor 6 is another type of sensor. It will also be appreciated that in the following discussion the relevant axis in the reference frame of the subject is the caudo-cranial axis, but the technique is also applicable to other axes in the reference frame of the subject.

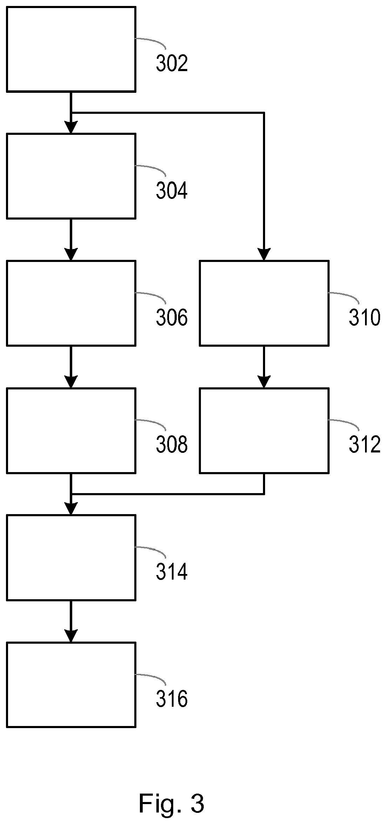

The flow chart in Fig. 3 shows an exemplary method of calibrating measurements from an accelerometer according to an embodiment. One or more of the steps of the method can be performed by the processing unit 12 in the apparatus 10, in conjunction with any of the sensor (accelerometer) 6, the ECG electrode arrangement 8, the memory unit 14, the interface circuitry 16 and the user interface 18 as appropriate. The processing unit 12 may perform the one or more steps in response to executing computer program code, that can be stored on a computer readable medium, such as, for example, the memory unit 14.

Initially the wearable device 4 is being worn or carried by the subject (e.g. it is worn on the subject using a chest band, or attached to the skin via an adhesive, etc.). The orientation of the wearable device 4 with respect to the subject is not known. In step 302 an ECG signal is acquired from each of the ECG electrode 9 pairs (ECG leads) in the ECG electrode arrangement 8. In steps 304 and 306 the QRS axis is determined as the direction perpendicular to the ECG lead that provides the smallest QRS complex, which defines the ventricular electrical activity during the cardiac cycle as sensed by the ECG lead, that is also unpolarised (i.e. the positive and negative component of the QRS complex in that ECG signal are similar in value). In step 308, which is optional, the angle of the QRS axis with respect to the caudo-cranial direction in the sagittal plane (i.e. a plane that divides the body into left and right parts) can be determined. In step 310 (which is optional) trends in the QRS complex, such as amplitude and polarity variation, between the ECG signals from the different ECG leads can be determined. In step 312 (which is performed in step 310 is performed) the sign (polarity) of the QRS axis can be determined so as to identify the anatomical left (or right) direction of the body. In step 314 a rotation matrix (or other relationship) is determined which allows a measurement axis of the accelerometer 6 to be 'aligned' with the caudo-cranial axis (and optionally also the medio-lateral left and forward direction of the body if steps 310 and 312 are performed). Finally in step 316 the determined rotation is used to translate accelerometer measurements into the reference frame of the subject (e.g. translate the measurements so that they are presented with respect to the caudo-cranial axis). Alternatively in step 316 a previously-established computational model in the reference frame of the subject that is used to analyse the accelerometer measurements (e.g. a set of rules or parameters that are used to determine the posture of the subject from accelerometer measurements) can be rotated into the reference frame of the accelerometer 6 so that the acceleration measurements can be evaluated using the rotated model.

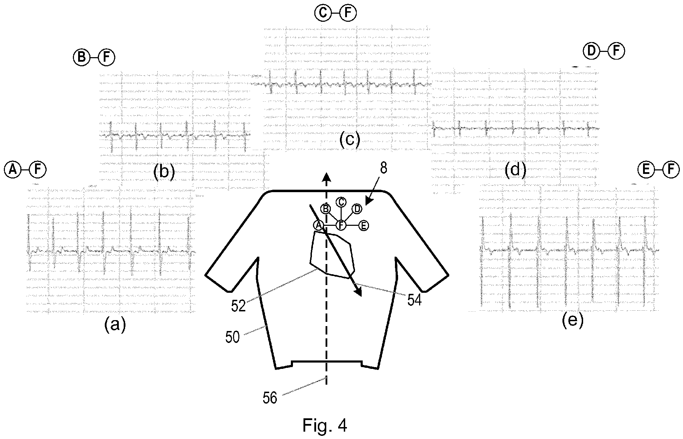

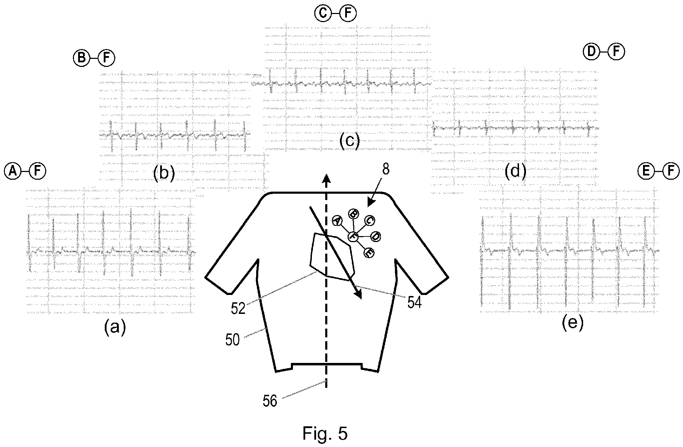

Thus in step 302 ECG signals are obtained from ECG leads that are oriented in multiple directions. Due to the arrangement of the ECG electrodes 9, the ECG leads are able to obtain ECG morphological data from different orientations with respect to the body of the subject. Figs. 4 and 5 are illustrations of the ECG electrode arrangement 8 of Fig. 2(c) in respective first and second orientations on the body of a subject and the corresponding ECG signals obtained by the ECG electrode arrangement 8.

In particular, Figs. 4 and 5 show the outline of part of a subject 50 (the torso) and an outline of the heart 52. The QRS axis of the heart 52 is shown by arrow 54, which for a typical subject 50 is generally oriented down and to the left in the reference frame of the subject 50. The caudo-cranial axis of the subject 50 is shown by arrow 56.

In Fig. 4 the ECG electrode arrangement 8 of Fig. 2(c) is being worn on the subject 50 in a first orientation that is such that the second ECG lead (comprised of ECG electrodes 9b and 9f) is the closest to being parallel with the QRS axis 54, and the fourth ECG lead (comprised of ECG electrodes 9d and 9f) is the closest to being perpendicular to the QRS axis 54. The ECG signals obtained by each of the first-fifth ECG leads are shown in the signal plots labelled (a)-(e) respectively in Fig. 4. The amplitude and time axes are not labelled in these plots, but the plots share the same amplitude and time scale.

It can be seen from the ECG signal plots in Fig. 4 that the ECG signal obtained by the fourth ECG lead has the smallest and least polarised QRS complex. This indicates that the virtual axis interconnecting ECG electrodes 9d and 9f (the fourth ECG lead) is the most orthogonal to the QRS axis 54.

In Fig. 5 the ECG electrode arrangement 8 of Fig. 2(c) is being worn on the subject 50 in a second orientation that is such that the first ECG lead (comprised of ECG electrodes 9a and 9f) and the fifth ECG lead (comprised of ECG electrodes 9e and 9f) are approximately parallel and anti-parallel to the QRS axis 54. In this second orientation the third ECG lead (comprised of ECG electrodes 9c and 9f) is the closest to being perpendicular to the QRS axis 54. The ECG signals obtained by each of the first-fifth ECG leads in the second orientation are shown in the signal plots labelled (a)-(e) respectively in Fig. 5. The amplitude and time axes are not labelled in these plots, but the plots share the same amplitude and time scale.

It can be seen from the ECG signal plots in Fig. 5 that the ECG signal obtained by the third ECG lead has the smallest and least polarised QRS complex. This indicates that the virtual axis interconnecting ECG electrodes 9c and 9f (the third ECG lead) is the most orthogonal to the QRS axis 54. Furthermore it can be noted that the QRS complex goes from positive to significantly negative when viewing the ECG signals in turn from the first ECG lead thorough to the fifth ECG lead. This information can be used to identify whether an axis perpendicular to the virtual axis of the third ECG lead (where the axis perpendicular to the virtual axis of the third ECG lead is considered to correspond to the QRS axis) is oriented towards the left or the right side of the accelerometer 6. This determines the medio-lateral left direction of the body of the subject 50, given that the QRS axis 54 most often points towards the left side of the body.

Once the ECG signals have been obtained, in step 304 the ECG signals are analysed to determine the maximum and minimum values of the voltage in each ECG signal. The aim is identify the ECG signal that corresponds to (or most corresponds to) the QRS complex, and thus the aim is to identify the ECG lead that is 'most isoelectric', i.e. the ECG lead that has the smallest QRS complex and for which the maximum value and the minimum value are most similar (in absolute terms).

After the most isoelectric ECG lead is determined in step 304 then in step 306 the QRS axis 54 can be identified by determining the direction that is perpendicular to the virtual axis associated with the most isoelectric ECG lead. For example, referring to Fig. 5, the third ECG lead is the most isoelectric ECG lead, and thus the direction of the QRS axis 54 can be determined to be perpendicular to the virtual axis that interconnects the ECG electrodes 9c and 9f.

It will be appreciated that depending on the number of ECG electrodes 9 in the ECG electrode arrangement 8 and their respective arrangement with respect to each other, it is possible for the actual QRS axis 54 is fall somewhere in between two adjacent ECG leads. Therefore in an alternative approach, the most isoelectric ECG lead can be identified in step 304, along with the next most isoelectric lead (which should be one of the two neighbouring ECG leads that has the second lowest QRS amplitude). An intermediate virtual axis that is considered to be orthogonal to the QRS axis 54 can be determined as a geometric mean (or a weighted mean) of the virtual axes of the two most isoelectric ECG leads. This intermediate virtual axis tends to provide a better approximation of a direction that is orthogonal to the QRS axis 54.

As an example of this alternative approach, consider in Fig. 4 that the fourth ECG lead is the most isoelectric ECG lead. The fourth ECG lead is oriented 45° from the fifth ECG lead. The QRS complex for the fourth ECG lead shows an amplitude of 400 millivolts, mV (from -200 mV to +200 mV) and the adjacent third ECG lead (which is oriented 90° from the fifth ECG lead) shows a QRS amplitude of 600 mV (from -200 mV to +400 mV). Therefore an intermediate virtual axis (i.e. corresponding to the direction of an ECG lead that would be orthogonal to the QRS axis 54) lies between third ECG lead and the fourth ECG lead, with an angle proportional to the amplitude of the QRS complexes of the two ECG leads. The angle of this intermediate virtual axis (denoted $\alpha^{ISO}$) could be determined as a weighted average of the angles of the two adjacent ECG leads with the lowest QRS amplitudes (the third ECG lead and the fourth ECG lead). According to the provided example, the fourth ECG lead has the lowest QRS complex while the third ECG lead has the second lowest QRS complex. Thus $\alpha^{ISO}$ can be given by:

$$\alpha^{ISO} = \alpha^{fc} + V^{fc} / (V^{fd} + V^{fc}) \cdot (\alpha^{fd} - \alpha^{fc}) \qquad (1)$$

where $\alpha^{fc}$ is the angle of the third ECG lead (that is comprised of ECG electrodes 9c and 9f) with respect to the measurement axis of the sensor 6 , $\alpha^{fd}$ is the angle of the fourth ECG lead (that is comprised of ECG electrodes 9d and 9f) with respect to the measurement axis of the sensor 6, $V^{fc}$ is the voltage of the QRS complex measured by the third ECG lead, and $V^{fd}$ is the voltage of the QRS complex measured by the fourth ECG lead. As noted above, the angle of the intermediate virtual axis ($\alpha^{ISO}$) represents the angle of the axis perpendicular to the QRS axis 54 in the frame of reference of the wearable device 4.

In step 308, which is optional, the direction of the caudo-cranial axis 56 in the frame of reference of the wearable device 4 can be determined by applying a rotation angle to the direction that is perpendicular to the most isoelectric ECG lead identified in step 306. Thus, the angle of the caudo-cranial axis 56 with respect to the wearable device 4 (denoted $\alpha^{CAUDO-CRANIAL}$) can be given by:

$$\alpha^{CAUDO-CRANIAL} = \alpha^{ISO} - \pi/2 - \alpha^{QRSax} \qquad (2)$$

where $\alpha^{QRSax}$ is the angle between the QRS axis 54 and the caudo-cranial axis 56. For a typical subject, $\alpha^{QRSax} = 38°$ left from the caudo-cranial axis. In some embodiments the value of $\alpha^{QRSax}$ can be personalised to the subject. In particular, the orientation of the heart, and thus the orientation of the QRS axis 54 in the reference frame of the subject, can vary from subject to subject, according to, for example, the weight of the subject, the body mass index, and/or other factors such as disease status (e.g. heart failure, cardiac myopathy, and structural heart disease) which can influence the angle between the QRS axis 54 and the caudo-cranial direction 56. In some embodiments information from 3-lead or 12-lead ECG examinations can be used to accurately determine the angle ($\alpha^{QRSax}$) of the QRS axis 54 in the reference frame of the subject.

In step 310 a change in the polarity of the QRS signal (QRS complex) through the set of ECG leads is determined. For example, considering the ECG electrode arrangement 8 in Fig. 2(c), if the QRS complex tends to become more negative or more positive when considering the first ECG lead through to the fifth ECG lead, then this can indicate whether the QRS axis 54 is oriented towards more towards the virtual axis defined by the first ECG lead or the fifth ECG lead. This information can be used to adjust the sign of the QRS axis angle ($\alpha^{QRSax}$) and to identify the anatomical left direction (step 312).

Once the caudo-cranial angle and the medio-lateral-left direction are determined in the frame of reference of the wearable device 4 (which is conceptually equivalent to the orientation (angle) of the wearable device 4 in the reference frame of the subject), in step 314 a rotation matrix (R) can be determined that enables acceleration measurements obtained in the reference frame of the accelerometer 6 to be rotated into the reference frame of the subject (i.e. with respect to the QRS axis 54, the caudo-cranial axis 56, or any other desired axis in the reference frame of the subject). Thus, the rotation matrix effectively enables the acceleration measurements to be processed so that each axis of the accelerometer 6 is measuring accelerations in the, e.g. caudo-cranial, medio-lateral and forward directions in the reference frame of the subject. It should be noted that the forward direction of the body can be assumed to be perpendicular and facing outwards from the wearable device 4. The rotation matrix R can be given by:

$$R = \begin{bmatrix} \cos(\alpha^{CAUDO-CRANIAL}), & \sin(\alpha^{CAUDO-CRANIAL}), & 0 \\ -\sin(\alpha^{CAUDO-CRANIAL}), & \cos(\alpha^{CAUDO-CRANIAL}), & 0 \\ 0, & 0, & 1 \end{bmatrix} \qquad (3)$$

Thus, the accelerometer 6 can be calibrated and oriented (within the plane of the wearable device 4) with respect to an anthropometric axis of the subject.

In some embodiments the ECG electrode arrangement 8 can also be used to determine one or more physiological characteristics of the subject, such as heart rate, heart rate variability, or pulse arrival time (which can be used to estimate blood pressure). In this case, only the ECG signal from the ECG lead that provides the largest maximum V is analysed to determine the values of the physiological characteristics. In the example of Fig. 5, this ECG lead would be that comprised of ECG electrodes 9e and 9f.

In some embodiments, the ECG signals obtained by the ECG electrode arrangement 8 can also be used to estimate the location of the wearable device 4 on the body of the subject 50 with respect to the heart 52 of the subject once the orientation of the wearable device 4 with respect to the subject 50 has been determined. This can be explained with reference to Fig. 5. In Fig. 5, the first ECG lead (comprised of ECG electrodes 9a and 9f) and the fifth ECG lead (comprised of ECG electrodes 9e and 9f) together form one straight line; in other words the first ECG lead is in the same direction as the fifth ECG lead. When the ECG electrode arrangement 8 is in the position and orientation on the body of the subject 50 shown in Fig. 5, the magnitude of $V^{fa}$ (which is the voltage of the QRS complex measured by the first ECG lead) will be smaller than the magnitude of $V^{ef}$ (from the fifth ECG lead), which can be seen in plots (a) and (e) of Fig. 5. In conjunction with having determined the orientation of the wearable device 4 on the subject 50 as described above, the magnitude of $V^{ef}$ being higher implies that ECG electrode 9a is further from the heart 52 than ECG electrode 9e, and thus the wearable device 4 is placed higher than the heart 52. The information on the location of the wearable device 4 could be used when the shape of the chest (including the shape of the breast in case the subject is female) is known. For example for a breast the angle of the skin with respect to the caudo-cranial axis 56 becomes smaller when the wearable device 4 is placed higher above the heart. The information on the angle of the skin (and thus wearable device 4) with respect to the caudo-cranial axis 56 can be used to more accurately determine the posture of the subject 50.

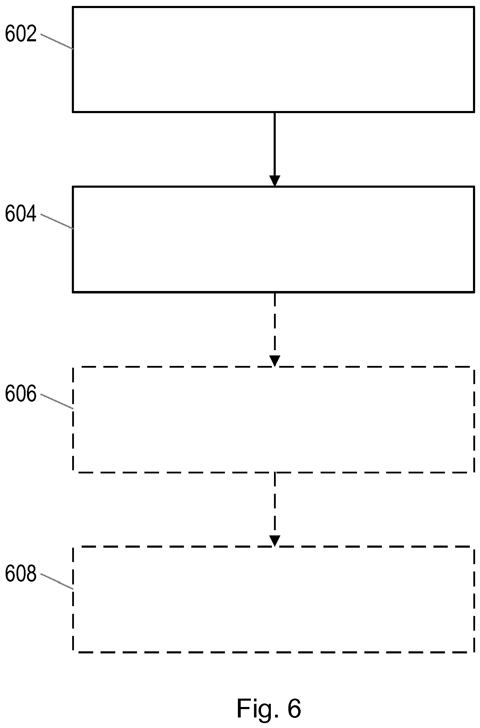

The flow chart in Fig. 6 illustrates a general method of calibrating a sensor 6 according to the techniques described herein. One or more of the steps of the method can be performed by the processing unit 12 in the apparatus 10, in conjunction with any of the sensor 6 (e.g. accelerometer), the ECG electrode arrangement 8, the memory unit 14, the interface circuitry 16 and the user interface 18 as appropriate. The processing unit 12 may perform the one or more steps in response to executing computer program code, that can be stored on a computer readable medium, such as, for example, the memory unit 14.

As noted above, the three or more ECG electrodes 9 of the ECG electrode arrangement 8 are in a predefined arrangement with respect to each other, and the sensor 6 has a measurement axis in a predefined orientation with respect to the ECG electrode arrangement 8. Information on the predefined arrangement of the ECG electrodes 9 with respect to each other can be stored in the memory unit 14. Information on the predefined orientation of the measurement axis of the sensor 6 with respect to the predefined ECG electrodes 9 can also be stored in the memory unit 14.

In a first step, step 602, the processing unit 12 determines an orientation of the ECG electrode arrangement 8 in the reference frame of the subject 50 based on ECG signals obtained by respective pairs of the ECG electrodes 9.

Step 602 can comprise a sub-step in which ECG signals are obtained using the ECG electrode arrangement 8 under the control of the processing unit 12. In this case, at least this sub-step of step 602 can be performed while the wearable device 4 is being carried or worn on the subject 50, and the processing unit 12 can obtain the ECG signals directly from the ECG electrode arrangement 8 (in embodiments where the apparatus 10 is part of the wearable device 4) or via interface circuitry 16 in the apparatus 10 and interface circuitry 20 in the wearable device 4 (in embodiments where the apparatus 10 is separate from the wearable device 4). Alternatively, step 602 can comprise a sub-step in which previously-obtained ECG signals are retrieved from a memory or storage (e.g. from memory unit 14), or are received from another component or device (e.g. received from wearable device 4) and are analysed. In this case, those ECG signals may have been obtained by the ECG electrode arrangement 8 under the control of the processing unit 12 or the wearable device 4 (if the wearable device 4 is separate from the apparatus 10). Also in this case, at least this sub-step of step 602 can be performed while the wearable device 4 is (still) being carried or worn on the subject 50 or it can be performed at a later time when the wearable device 4 is no longer being worn or carried by the subject 50 (e.g. in embodiments where the analysis of the ECG signals and calibrating the sensor 6 is performed offline).

In either case, respective ECG signals are obtained for each of the ECG leads defined in the ECG electrode arrangement 8. The ECG signals can all relate to the same time period (to make sure that there is no change in orientation of the wearable device 4 between ECG measurements). Alternatively, if the orientation of the wearable device 4 is not likely to change over time, i.e. if the orientation of the wearable device 4 with respect to the subject 50 does not change once the wearable device 4 is being worn, e.g. if the wearable device 4 is attached to the subject 50 via a chest strap or band, then the respective ECG signals obtained for each of the ECG leads do not all have to relate to the same time period.

In step 602 the processing unit 12 analyses the ECG signals to identify one or more pairs of ECG electrodes 9 (i.e. one or more ECG leads) that exhibit or best exhibit a particular ECG signal characteristic, such as a QRS complex or R-peak. The orientation of the identified ECG lead in the ECG electrode arrangement 8 is known, as is the orientation of a reference axis relating to the heart 52 of the subject 50 (e.g. a QRS axis) along which the particular ECG characteristic is strongest or weakest (as appropriate), which means that the orientation of the ECG electrode arrangement 8 in the reference frame of the subject 50 can be determined.

Thus, in some embodiments, the processing unit 12 determines the orientation of the ECG electrode arrangement 8 by identifying a pair of ECG electrodes 9 in the ECG electrode arrangement 8 for which the ECG signal obtained by the ECG electrode pair meets a criterion relating to the predefined ECG signal characteristic, and determining the orientation of the ECG electrode arrangement 8 with respect to the reference frame of the subject 50 based on a first virtual axis defined by the ECG electrodes 9 in the identified ECG electrode pair and the relation between the reference axis and the predefined ECG signal characteristic.

In alternative embodiments, which may be useful where greater accuracy is desired for the calibration of the sensor 6, where the ECG electrode arrangement 8 defines a small number of ECG leads and/or where the differences between the orientations of ECG leads defined by the ECG electrode arrangement 8 are relatively large, it can be assumed that the reference axis lies between the virtual axes defined by two adjacent pairs of ECG electrodes 9, and a refined virtual axis should be determined. Thus the processing unit 12 determines the orientation of the ECG electrode arrangement 8 by identifying two adjacent pairs of ECG electrodes 9 in the ECG electrode arrangement 8 for which the respective ECG signals best meet a criterion relating to the predefined ECG signal characteristic, and determining a first virtual axis (corresponding to the reference axis) as an axis between the virtual axes of the two adjacent ECG electrode pairs. The orientation of the ECG electrode arrangement 8 with respect to the reference frame of the subject 50 is then determined based on the first virtual axis and the relation between the reference axis and the predefined ECG signal characteristic.

In either of the above sets of embodiments, the predefined ECG signal characteristic can be the QRS complex and the criterion is met or best met by the ECG signal that has the smallest QRS complex, and/or the smallest difference in maximum voltage amplitude and minimum voltage amplitude. In these embodiments, the reference axis in the reference frame of the subject 50 can be the QRS axis 54 of the heart 52 of the subject 50. In determining the orientation of the wearable device 4 in the reference frame of the subject 50, the first virtual axis is considered to be perpendicular or substantially perpendicular to the QRS axis.

In either of the above sets of embodiments, the predefined ECG signal characteristic can be the R-peak and the criterion is met or best met by the ECG signal that has the largest R-peak, and/or the largest difference in maximum voltage amplitude and minimum voltage amplitude. In these embodiments, the reference axis in the reference frame of the subject 50 can be the QRS axis 54 of the heart 52 of the subject 50. In determining the orientation of the wearable device 4 in the reference frame of the subject 50, the first virtual axis is considered to be parallel or substantially parallel to the QRS axis.

Once the orientation of the wearable device 4/ECG electrode arrangement 8 in the reference frame of the subject 50 has been determined in step 602, then in step 604 the processing unit 12 determines the relationship between the measurement axis of the sensor 6 and the reference frame of the subject 50. This relationship is determined based on the orientation of the ECG electrode arrangement 8 in the reference frame of the subject determined in step 602 and the predefined (known) orientation of the measurement axis of the sensor 6 with respect to the ECG electrode arrangement 8. It will be appreciated that in some embodiments this step can comprise applying the predefined orientation of the measurement axis of the sensor 6 with respect to the ECG electrode arrangement 8 (which can be expressed as one or more angles) to the orientation determined in step 602. The relationship may be determined as a rotation between the reference frame of the subject 50 (or an axis in the reference frame of the subject 50, such as the QRS axis 54 or the caudo-cranial axis 56). The rotation may be expressed as one or more rotation angles with respect to one or more axes in the reference frame of the subject 50 or in the reference frame of the sensor 6, or as a rotation matrix.

In the embodiments above where the reference axis is the QRS axis 54, the method can further comprise the processing unit 12 determining the polarity of the ECG signal obtained by the ECG electrode pair that best meets the criterion. The polarity indicates the direction of the QRS axis in the reference frame of the ECG electrode arrangement 8, and thus the processing unit 12 can use the polarity to determine the orientation of the ECG electrode arrangement 8 with respect to the left and right side of the subject. In these embodiments, in step 604 the processing unit 12 can then determine the relationship between the measurement axis of the sensor 6 and the reference frame of the subject based on the determined orientation of the ECG electrode arrangement 8 with respect to the left and right side of the subject.

In some cases the orientation of the reference axis in the reference frame of the subject 50 may differ from subject to subject. That is, the orientation of the QRS axis 54 with respect to the caudo-cranial axis 56 for one subject may differ to that orientation for another subject. Therefore, in step 601, the orientation of the wearable device 4/ECG with respect to the reference frame of the subject 50 can take into account one or more physiological characteristics of the subject and/or clinical information on the subject, such as an orientation of the QRS axis 54 in the subject 50 or the orientation of the subject's heart 52. In other embodiments, the orientation of the QRS axis 54 can be assumed to have a standard orientation with respect to the body of the subject 50, e.g. 38° left from the caudo-cranial axis 56.

In some cases the orientation of the wearable device 4 can be affected by the shape of the chest of the subject 50 (particularly where the wearable device 4 is carried or worn on a breast and/or the subject 50 is overweight), and so in some embodiments the processing unit 12 can in step 604 determine the relationship between the measurement axis of the sensor 6 and the reference frame of the subject based on a shape of the part of the body of the subject on which the wearable device is to be worn. In particular, when the wearable device 4 is worn on the upper chest, the shape of the chest could be taken into account to identify the angle between the sagittal plane, on which the heart resides, and the surface of the body where the wearable device 4 is worn and the ECG signals are measured. Thus, the final orientation of the sagittal plane could be tilted by an angle proportional to the curvature of the skin with respect to the direction of the caudo-cranial axis or other axis in the reference frame of the subject. As another example, when the wearable device 4 is worn mid-clavicular at the level of the lower left rib, the shape of the body at this location could be taken into account to identify the angles between the surface of the body where the wearable device 4 is worn and the ECG signals are measured and each of the sagittal plane, the frontal plane (i.e. a plane that divides the body into front and back parts) and the transverse plane (i.e. a plane that divides the body into top and bottom parts). Thus, the final orientation of the sagittal plane, frontal plane and the transverse plane could be tilted by an angle proportional to the curvature of the skin with respect to the direction of the caudo-cranial axis or other axis in the reference frame of the subject.

As noted above, the relationship determined in step 604 can be a rotation from one reference frame (i.e. of the sensor 6 or the subject) to the other (i.e. of the subject or the sensor 6). This rotation can be used to rotate measurements obtained by the sensor 6 in the reference frame of the sensor 6 into the reference frame of the subject 50. Alternatively the rotation can be used to rotate one or more parameters and/or rules defined in the reference frame of the subject 50 into the reference frame of the sensor 6 so that the measurements from the sensor 6 can be evaluated using the rotated parameter(s) and/or rule(s).

For example, the one or more parameters and/or rules can be used for determining the posture of the subject 50 from acceleration measurements, with a first rule that if the acceleration is in a vertical direction in the reference frame of the subject 50 (e.g. along a z-axis) then the subject 50 is standing up, a second rule that if the acceleration is in a horizontal (forward) direction in the reference frame of the subject 50 (e.g. along a y-axis) then the subject 50 is lying supine, a third rule that if the acceleration is in an horizontal (lateral) direction in the reference frame of the subject 50 (e.g. along a x-axis) then the subject 50 is lying on their side. The rotation determined in step 604 can be used to rotate the rules into the reference frame of the accelerometer 6.

In some embodiments, the method in Fig. 6 can further comprise a step 606 in which the processing unit 12 acquires or obtains measurements from the sensor 6. The sensor 6 measures the subject 50 while the wearable device 4 is being carried or worn by the subject 50. The measurements of the subject relate to the subject during a time period (which can be the same time period that the ECG signals relate to). In step 606 the processing unit 12 can obtain the measurements directly from the sensor 6 (in embodiments where the apparatus 10 is part of the wearable device 4) or via interface circuitry 16 in the apparatus 10 and interface circuitry 20 in the wearable device 4 (in embodiments where the apparatus 10 is separate from the wearable device 4). Alternatively, step 606 can comprise the processing unit 12 obtaining or retrieving previously-obtained sensor measurements from a memory or storage (e.g. from memory unit 14), or are received from another component or device (e.g. received from wearable device 4). In this case, those measurements may have been obtained by the sensor 6 under the control of the processing unit 12 or the wearable device 4 (if the wearable device 4 is separate from the apparatus 10).

In some embodiments, in step 608 the rotation determined in step 604 can be applied to the acquired measurements from the sensor 6 to rotate the acquired measurements into the reference frame of the subject 50. The rotated measurements can then be evaluated using one or more parameters and/or rules defined with respect to the reference frame of the subject 50. For example the one or more parameters and/or rules may be for determining the posture of the subject.

In alternative embodiments of step 608 the rotation determined in step 604 can be applied to one or more parameters and/or rules to rotate the one or more parameters and/or rules into the reference frame of the sensor 6. The acquired measurements can then be evaluated using the rotated one or more parameters and/or rules. For example the one or more parameters and/or rules may be for determining the posture of the subject.

Thus the above techniques provide that at least some of the limitations with existing techniques for calibrating a sensor are addressed. In particular a wearable device 4 comprising a sensor 6 can be placed in any orientation on the subject 50 and it is possible to determine the orientation and thus calibrate the sensor 6 to account for that orientation. Moreover, determining an orientation of a QRS axis 54 of the heart 52 of the subject 50 with respect to the wearable device 4, (optionally determining an orientation of a caudo-cranial axis 56 of the subject 50), and calibrating the sensor 6 of the wearable device 4 based on this determined orientation provides a more reliable and more accurate calibration of the sensor 6. Moreover, the calibration of the sensor 6 can be achieved without having any prior knowledge of the orientation of the wearable device 4, only the orientation and arrangement of the ECG electrodes 9 in the ECG electrode arrangement 8, and the orientation of the ECG electrode arrangement 8 with respect to a measurement axis of the sensor 6. The calibration can also be achieved without requiring the subject 50 to undertake any specific activity or movement (e.g. walking upright).

There is thus provided herein an improved technique for calibrating a sensor, which addresses the limitations associated with the existing techniques.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An apparatus for calibrating a sensor of a wearable device to a reference frame of a subject on which the wearable device is to be worn, the wearable device comprising an electrocardiogram, ECG, electrode arrangement comprising three or more ECG electrodes that are in a predefined arrangement with respect to each other, and the sensor having a measurement axis in a predefined orientation with respect to the ECG electrode arrangement, the apparatus comprising a processing unit configured to:

   determine an orientation of the ECG electrode arrangement in the reference frame of the subject based on ECG signals obtained by respective pairs of the ECG electrodes; and
   determine a relationship between the measurement axis of the sensor and the reference frame of the subject based on the determined orientation of the ECG electrode arrangement in the reference frame of the subject and the predefined orientation of the measurement axis with respect to the ECG electrode arrangement.

2. The apparatus as claimed in claim 1, wherein the reference frame of the subject includes a reference axis relating to the heart of the subject, wherein the reference axis is related to a predefined ECG signal characteristic, and wherein the processing unit is configured to determine the orientation of the ECG electrode arrangement by:

   identifying an ECG electrode pair in the ECG electrode arrangement for which the ECG signal obtained by the ECG electrode pair meets a criterion relating to the predefined ECG signal characteristic; and
   determining the orientation of the ECG electrode arrangement with respect to the reference frame of the subject based on a first virtual axis defined by the ECG electrodes in the identified ECG electrode pair and the relation between the reference axis and the predefined ECG signal characteristic.

3. The apparatus as claimed in claim 1, wherein the reference frame of the subject includes a reference axis relating to the heart of the subject, wherein the reference axis is related to a predefined ECG signal characteristic, and wherein the processing unit is configured to determine the orientation of the ECG electrode arrangement by:

   identifying two adjacent ECG electrode pairs in the ECG electrode arrangement for which the ECG signals obtained by the two adjacent ECG electrode pairs best meet a criterion relating to the predefined ECG signal characteristic;
   determining a first virtual axis as a virtual axis between a virtual axis defined by the ECG electrodes in a first one of the identified ECG electrode pairs and a virtual axis defined by the ECG electrodes in the other one of the identified ECG electrode pairs; and
   determining the orientation of the ECG electrode arrangement with respect to the reference frame of the subject based on the first virtual axis and the relation between the reference axis and the predefined ECG signal characteristic.

4. The apparatus as claimed in claim 2 or 3, wherein the predefined ECG signal characteristic is the QRS complex and the criterion is met by the ECG signal that has the smallest QRS complex; or the smallest difference in maximum voltage amplitude and minimum voltage amplitude.

5. The apparatus as claimed in claim 2 or 3, wherein the predefined ECG signal characteristic is the R-peak and the criterion is met by the ECG signal that has the largest R-peak; or the largest difference in maximum voltage amplitude and minimum voltage amplitude.

6. The apparatus as claimed in any of claims 2-5, wherein the orientation of the reference axis in the reference frame of the subject is dependent on one or more physiological characteristics of the subject and/or clinical information on the subject.

7. The apparatus as claimed in any of claims 1-6, wherein the processing unit is further configured to determine the relationship between the measurement axis of the sensor and the reference frame of the subject based on a shape of the part of the body of the subject on which the wearable device is to be worn.

8. The apparatus as claimed in any of claims 1-7, wherein the determined relationship between the measurement axis of the sensor and the reference frame of the subject is a rotation required to (i) rotate measurements obtained by the sensor in the reference frame of the sensor into the reference frame of the subject and/or (ii) rotate one or more parameters or rules defined in the reference frame of the subject into the reference frame of the sensor.

9. The apparatus as claimed in claim 8, wherein the processing unit is configured to:

   acquire measurements from the sensor;
   apply the rotation to the acquired measurements to rotate the acquired measurements into the reference frame of the subject; and
   evaluate the rotated measurements to determine the posture of the subject using one or more parameters and/or rules defined with respect to the reference frame of the subject.

10. The apparatus as claimed in claim 8, wherein the processing unit is configured to:

    acquire measurements from the sensor;
    apply the rotation to one or more parameters and/or rules defined with respect to the reference frame of the subject to rotate the one or more parameters and/or rules into the reference frame of the sensor; and
    evaluate the acquired measurements to determine the posture of the subject using the rotated one or more parameters and/or rules.

11. A system for calibrating a sensor of a wearable device to a reference frame of a subject on which the wearable device is worn, the system comprising:

    the apparatus as claimed in any of claims 1-10; and
    the wearable device comprising the ECG electrode arrangement and the sensor having the measurement axis in the predefined orientation with respect to the ECG electrode arrangement.

12. A method of calibrating a sensor of a wearable device to a reference frame of a subject on which the wearable device is to be worn, the wearable device comprising an electrocardiogram, ECG, electrode arrangement comprising three or more ECG electrodes that are in a predefined arrangement with respect to each other, and the sensor having a measurement axis in a predefined orientation with respect to the ECG electrode arrangement, the method comprising:

    determining an orientation of the ECG electrode arrangement in the reference frame of the subject based on ECG signals obtained by respective pairs of the ECG electrodes; and
    determining a relationship between the measurement axis of the sensor and the reference frame of the subject based on the determined orientation of the ECG electrode arrangement in the reference frame of the subject and the predefined orientation of the measurement axis with respect to the ECG electrode arrangement.

13. The method as claimed in claim 12, wherein the determined relationship between the measurement axis of the sensor and the reference frame of the subject is a rotation required to (i) rotate measurements obtained by the sensor in the reference frame of the sensor into the reference frame of the subject, and the method further comprises:

    acquiring measurements from the sensor;
    applying the rotation to the acquired measurements to rotate the acquired measurements into the reference frame of the subject; and
    evaluating the rotated measurements to determine the posture of the subject using one or more parameters and/or rules defined with respect to the reference frame of the subject.

14. The method as claimed in claim 12, wherein the determined relationship between the measurement axis of the sensor and the reference frame of the subject is a rotation required to rotate one or more parameters or rules defined in the reference frame of the subject into the reference frame of the sensor, and the method further comprises:

    acquiring measurements from the sensor;
    applying the rotation to one or more parameters and/or rules defined with respect to the reference frame of the subject to rotate the one or more parameters and/or rules into the reference frame of the sensor; and
    evaluating the acquired measurements to determine the posture of the subject using the rotated one or more parameters and/or rules.

**15.** A computer program product comprising a computer readable medium, the computer readable medium having a computer readable code embodied therein, the computer readable code being configured such that, on execution by a suitable computer or processing unit, the computer or processing unit is caused to perform the method as claimed in any of claims 12-14.

Fig. 1

Fig. 2

Fig. 3

Fig. 4

Fig. 5

Fig. 6

Europäisches
Patentamt

European
Patent Office

Office européen
des brevets

# EUROPEAN SEARCH REPORT

Application Number

EP 19 18 9368

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2017/191036 A1 (KONINKLIJKE PHILIPS NV [NL]) 9 November 2017 (2017-11-09) * page 4, line 18 - line 24 * * page 5, line 1 - line 3 * * figure 2 * ----- | 1-15 | INV. A61B5/0402 |
| A | US 2006/253043 A1 (ZHANG YI [US] ET AL) 9 November 2006 (2006-11-09) * paragraphs [0040], [0092] * ----- | 4,5 | |
| A | US 2019/150795 A1 (LU BIN [US]) 23 May 2019 (2019-05-23) * paragraph [0019] * ----- | 7 | |

TECHNICAL FIELDS
SEARCHED (IPC)

A61B

The present search report has been drawn up for all claims

| Place of search | Date of completion of the search | Examiner |
|---|---|---|
| The Hague | 24 January 2020 | Vanderperren, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 18 9368

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2020

| Patent document cited in search report | | | Publication date | Patent family member(s) | | | Publication date |
|---|---|---|---|---|---|---|---|
| WO 2017191036 | A1 | | 09-11-2017 | BR | 112018072580 | A2 | 19-02-2019 |
| | | | | CN | 109069004 | A | 21-12-2018 |
| | | | | EP | 3451901 | A1 | 13-03-2019 |
| | | | | JP | 2019516454 | A | 20-06-2019 |
| | | | | US | 2019133488 | A1 | 09-05-2019 |
| | | | | WO | 2017191036 | A1 | 09-11-2017 |
| US 2006253043 | A1 | | 09-11-2006 | NONE | | | |
| US 2019150795 | A1 | | 23-05-2019 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017191036 A